# EUROPEAN PATENT SPECIFICATION

(11) **EP 2 731 529 B1**
(45) Date of publication and mention of the grant of the patent: **05.09.2018**
(21) Application number: 12811731.4
(22) Date of filing: 09.07.2012
(51) Int. Cl.: A61B 18/02, A61B 1/018

(54) **FOCAL ABLATION ASSEMBLY**
FOKALE ABLATIONSANORDNUNG
ENSEMBLE D'ABLATION FOCALE

(30) Priority: 11.07.2011 US 201113180443; 11.07.2011 US 201113180450
(43) Date of publication of application: 21.05.2014
(73) Proprietor: Pentax of America, Inc., Montvale, NJ 07645 (US)
(72) Inventor: WU, Patrick, P., San Carlos, CA 94070 (US); ICO, Cesar, A., San Francisco, CA 94112 (US); WILLIAMS, Richard, S., Redwood City, CA 94062 (US)
(74) Representative: Clark, Jane Anne
(86) International application number: PCT/US2012/045952
(87) International publication number: WO 2013/009700

(56) References cited:
- RU-C2- 2 178 999
- US-A1- 2002 143 323
- US-A1- 2007 299 433
- US-A1- 2008 058 591
- US-A1- 2009 182 319
- US-A1- 2009 299 355
- US-A1- 2010 057 065
- US-A1- 2010 057 065
- US-A1- 2010 130 970
- US-A1- 2010 130 970
- US-B1- 6 283 959
- US-B1- 6 355 029

## Description

### BACKGROUND OF THE INVENTION

Throughout the GI tract in the human body there are focal lesions of unwanted or unhealthy tissue that physicians desire to remove or ablate *in situ.* Examples of these lesions include 'islands' of intestinal metaplasia and dysplasia in the esophagus or 'flat' polyps in the colon. Removal of these tissues through techniques such as Endoscopic Mucosal Resection (EMR) may create unwanted complications such as bleeding and current ablative modalities such as Argon Plasma Coagulation (APC) and Radio Frequency Ablation (RFA) suffer from a variety of drawbacks. Furthermore, existing cryoablation technologies, which spray the cryogen directly onto the body lumen do not adequately allow control of the energy dosage.

US 2008/0058591 describes tissue visualisation devices. Figures 31A to 31C show an imaging system having an imaging hood within which is mounted a probe having a distal end effector which may be configured as an ablation probe utilising radio frequency energy, microwave energy, ultrasound energy, laser energy or cryo-ablation. The end effector may have several electrodes for detecting or mapping electrical signals transmitted through the underlying tissue.

US 2010/0130970 describes a device for treating esophageal target tissue comprising a catheter, a balloon and a refrigerant delivery device. The catheter has a distal portion and a refrigerant delivery lumen. The balloon is mounted to and the refrigerant delivery device is coupled to the distal portion. The refrigerant delivery device comprises a chamber with the refrigerant delivery lumen opening into the chamber, a refrigerant delivery opening fluidly coupled to the balloon interior and a distribution passageway fluidly coupling the chamber and the refrigerant delivery opening. A refrigerant is deliverable through the refrigerant delivery lumen into the chamber through the distribution passageway through the refrigerant delivery opening and into the balloon interior so as to place the balloon into an expanded cooled state so that the balloon can press against and cool esophageal target tissue.

### BRIEF SUMMARY OF THE INVENTION

The present invention is set out in the appended claims. Described herein is an example of a focal ablation assembly used with an endoscope comprising an endoscopic tube having proximal and distal ends and defining a channel extending between the proximal and distal ends. The focal ablation assembly comprises a cryogenic catheter, a balloon and a reinforcing element. The cryogenic catheter is placeable within the channel. The cryogenic catheter defines a catheter lumen and has a distal end placeable at the distal end of the endoscopic tube. The balloon is mountable to at least one of the distal end of the endoscopic tube and the distal end of the catheter. The balloon extends distally of both of the distal ends of the endoscopic tube and the cryogenic catheter. The reinforcing element at least partially defines the shape of the balloon in the expanded state. The balloon defines a balloon volume when in the expanded state. The balloon comprises a thermally conductive therapeutic region, the thermally conductive therapeutic region providing effectively no thermal insulation. In some examples the therapeutic region comprises a flexible, tissue-conformable therapeutic region. In some examples the thermal conductivity of the balloon is greater at the therapeutic region than at a portion of the remainder of the balloon. In some examples the focal ablation assembly comprises a delivery catheter extending along the channel with a distal portion fluidly coupled to the balloon interior, whereby refrigerant can be introduced into the balloon interior and towards the therapeutic region by the delivery catheter. In some examples the reinforcing elements are formed integrally with the balloon. In some examples the reinforcing elements comprise at least two spaced-apart support wires extending at least part way along the balloon interior. In some examples the reinforcing elements cause the balloon to have a flattened cross-sectional shape in the expanded state.

An example of a focal ablation system comprises an endoscope and a focal ablation assembly. The endoscope comprises an endoscopic tube having proximal and distal ends and defines a channel extending between the proximal and distal ends. The focal ablation assembly comprises a cryogenic catheter, a balloon, and a reinforcing element. The cryogenic catheter is located within the channel and defines a catheter lumen. The cryogenic catheter has a distal end at the distal end of the endoscopic tube. The balloon is mounted to the distal end of the catheter with the balloon extending distally of both of the distal ends of the endoscopic tube and the cryogenic catheter. The balloon is placeable in collapsed and expanded states and has a balloon interior. The reinforcing element at least partially defines the shape of the balloon in the expanded state. The balloon defines a balloon volume when in the expanded state. The balloon comprises a flexible, tissue-conformable, thermally conductive therapeutic region, the thermally conductive therapeutic region providing effectively no thermal insulation. Some examples of the focal ablation system include a delivery catheter extending along the channel and having a distal portion fluidly coupled to the balloon interior, whereby refrigerant can be introduced into the balloon interior and towards the therapeutic region by the refrigerant delivery catheter. Some examples of the focal ablation system include an exhaust lumen within at least one of (1) the channel of the endoscopic tube, and (2) the catheter lumen.

Another example of a focal ablation assembly is used with an endoscope comprising an endoscopic tube having proximal and distal ends and defining a channel extending between the proximal and distal ends. The focal ablation assembly comprises a cryogenic catheter and a cap. The cryogenic catheter is placeable within the channel. The cryogenic catheter defines a catheter lumen and has a distal end placeable at the distal end of the endoscopic tube. The cap is mountable to at least one of the distal end of the endoscopic tube and the distal end of the cryogenic catheter. The cap extends distally of both of the distal ends. The cap comprises a material which substantially maintains its shape during use while not causing tissue trauma. The cap defines a limited area therapeutic region. A cap volume is defined by a wall of the cap and the therapeutic region. The therapeutic region is a chosen one of an open therapeutic region and a covered, optically transparent therapeutic region providing effectively no thermal insulation. In some examples the cap is made of a flexible, soft polymer material. In some examples the cap has a distal end and the therapeutic region is at the distal end of the cap while in other examples the cap has a sidewall and the therapeutic region is at the sidewall. In some examples the focal ablation assembly includes a delivery catheter extending along the channel and has a distal portion fluidly coupled to the cap volume, whereby refrigerant can be introduced into the cap volume and towards the therapeutic region by the delivery catheter. In some examples the focal ablation assembly includes a balloon extending from the distal end of the cryogenic catheter within the therapeutic region so that the refrigerant can be introduced into the balloon within the cap volume by the delivery catheter.

Other features, aspects and advantages of the present invention can be seen on review the figures, the detailed description, and the claims which follow.

### BRIEF DESCRIPTION OF THE DRAWINGS

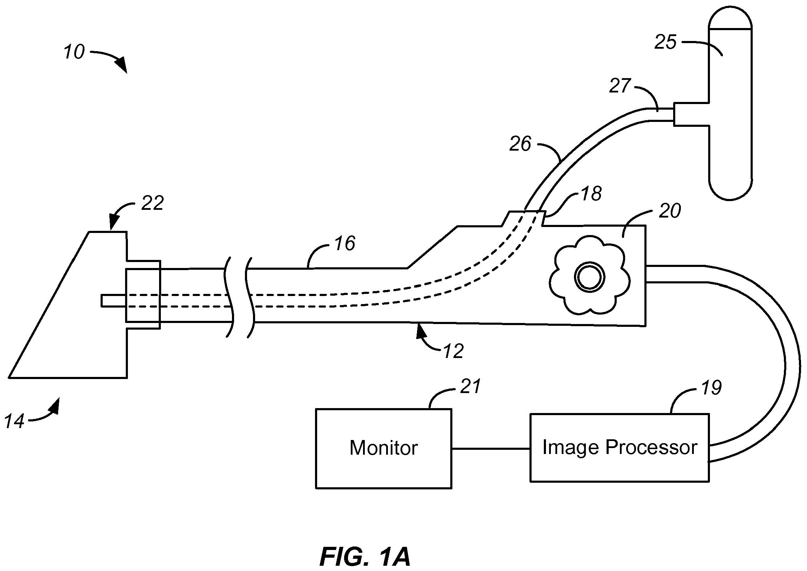

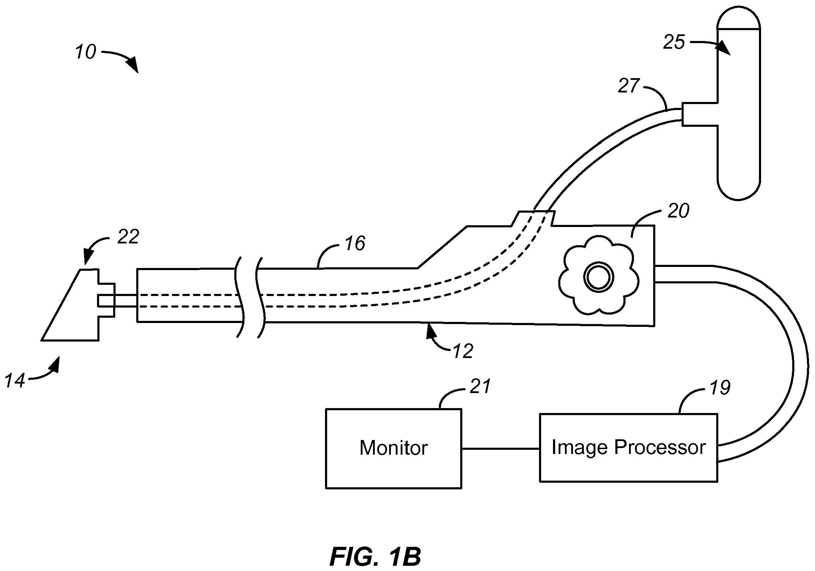

Figs. 1A and 1B are overall views showing focal ablation systems.
Figs. 1A-11 are directed to a first type of a focal ablation system.
Fig. 2 is a simplified enlarged cross-sectional view of the distal end of one example of the system of Fig. 1A showing a cryogenic catheter passing through the channel of an endoscopic tube with a cap mounted to the distal end of the endoscopic tube, the cap having a thermally conductive therapeutic region located at the distal end of the cap.
Fig. 3 is a view similar to that of Fig. 2 of an alternative example of the system of Fig. 1A in which the therapeutic region is along a sidewall of the cap.
Fig. 4 is a view similar to that of Fig. 2 wherein the cap is mounted to the distal end of the cryogenic catheter instead of the endoscopic tube as shown in Fig. 1B.
Figs. 5A-5C are simplified cross-sectional views of examples of three different caps in which the cross-sectional area of the therapeutic region at the distal end of each cap is different while the cross-sectional area of the endoscopic tube to which it is mounted remains the same.
Fig. 6 illustrates an example in which the therapeutic region has a convex outer surface.
Figs. 7A, 8A and 9A are simplified end views showing caps having round, generally oval and rectangular cross-sectional shapes, respectively, each mounted to the distal end of an endoscope, each Fig. illustrating a conventional endoscope having, in this example, a camera, two lights for illumination and a working channel.
Figs. 7B, 8B and 9B show the caps of Figs. 7A, 8A and 9A having distal ends oriented perpendicular to the centerlines of the respective caps.
Figs. 7C, 8C and 9C show the caps of Figs. 7A, 8A and 9A having distal ends oriented obliquely to the centerlines of the respective caps.
Fig. 10 shows an example of a cap similar to the cap of Fig. 2 but in which the cap has an extension defining an exhaust lumen coaxial with the endoscopic tube.
Figs. 11 -15 show balloon-type of focal ablation systems according to the invention.

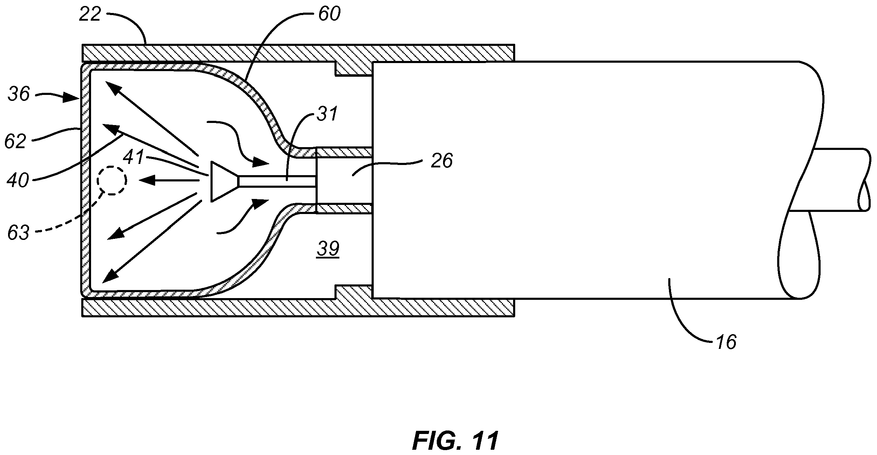

Fig. 11 shows an example of a balloon type focal ablation system in which an elastomeric balloon is mounted to the distal end of the cryogenic catheter and expands within the cap, a portion of the balloon forming a barrier to the refrigerant along the therapeutic region at the distal end of the cap.

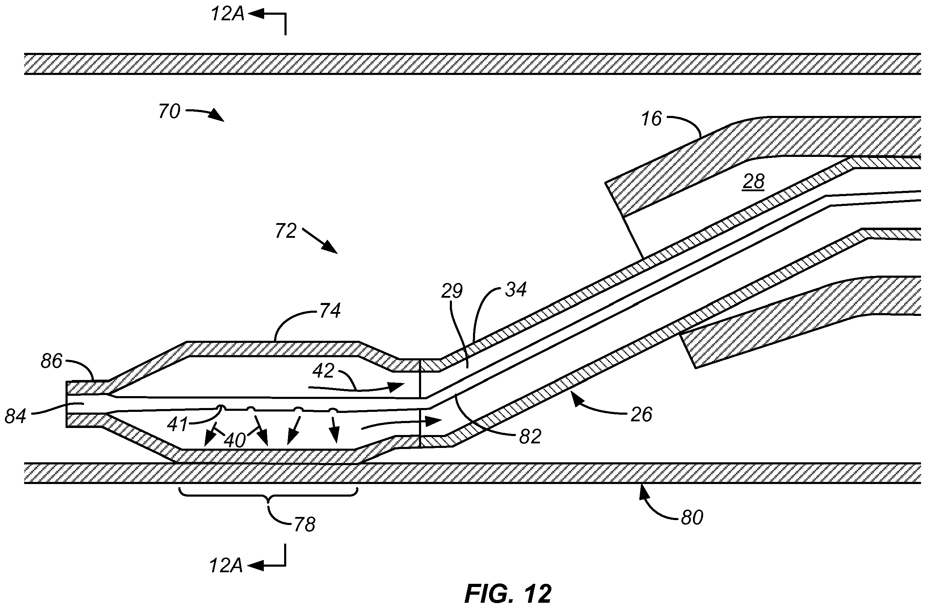

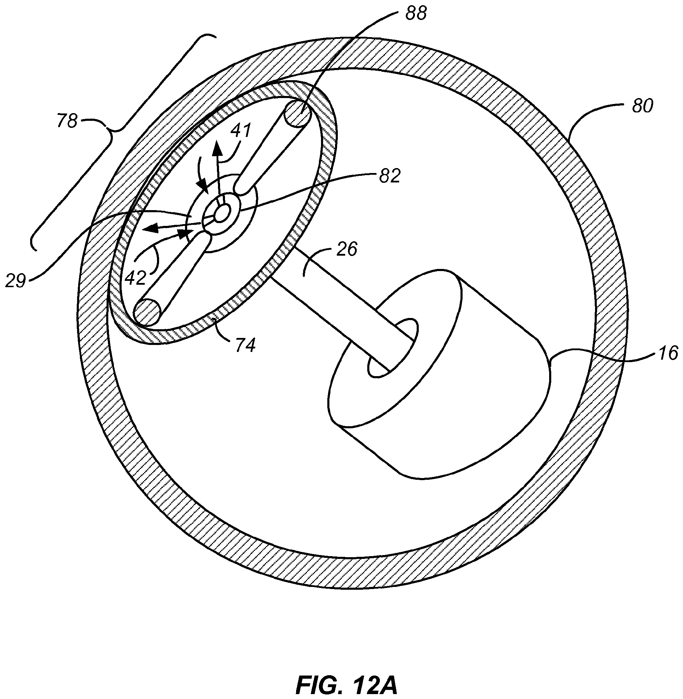

Figs. 12 and 12A are simplified side and end cross-sectional views of a balloon type focal ablation system in which a balloon type focal ablation assembly is used with an endoscopic tube, the balloon being mounted to the distal end of a cryogenic catheter, the cryogenic catheter passing through the channel of the endoscopic tube, the therapeutic region being along a sidewall of the balloon. Fig. 12A shows the use of reinforcing elements within the balloon to cause the balloon to have a flattened or oval cross-sectional shape to better conform to the sidewall of the body lumen.

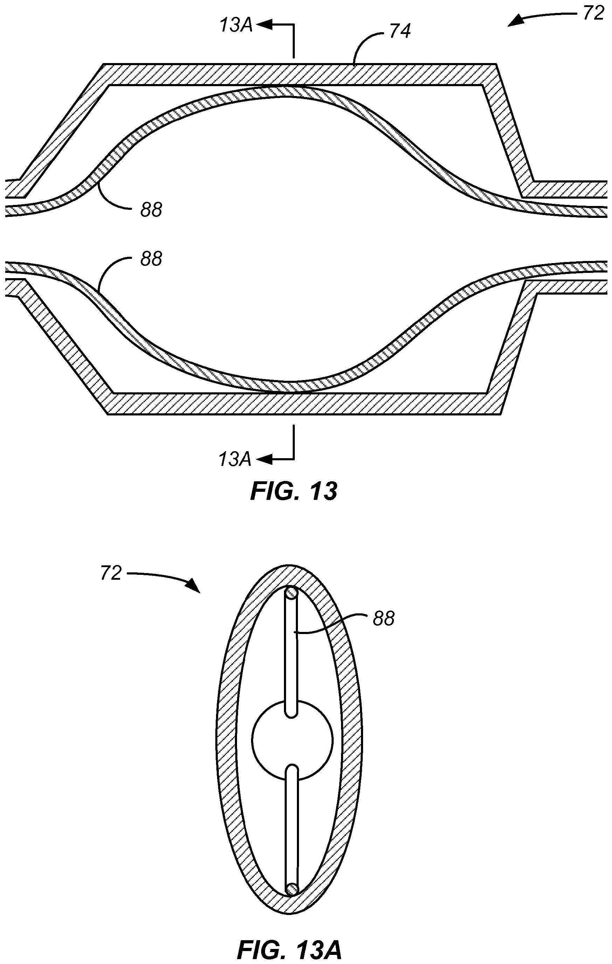

Figs. 13 and 13A are simplified side and end cross-sectional views of a focal ablation balloon and reinforcing elements similar to those of Figs. 12 and 12A.

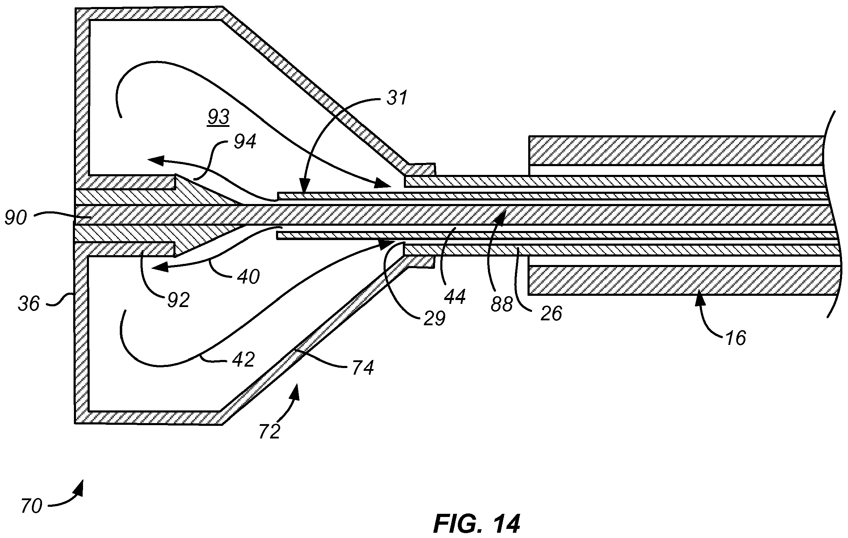

Fig. 14 illustrates another example of a balloon type focal ablation system in which the distal end of a reinforcing element extends through the delivery catheter and is secured to the distal portion of the balloon.

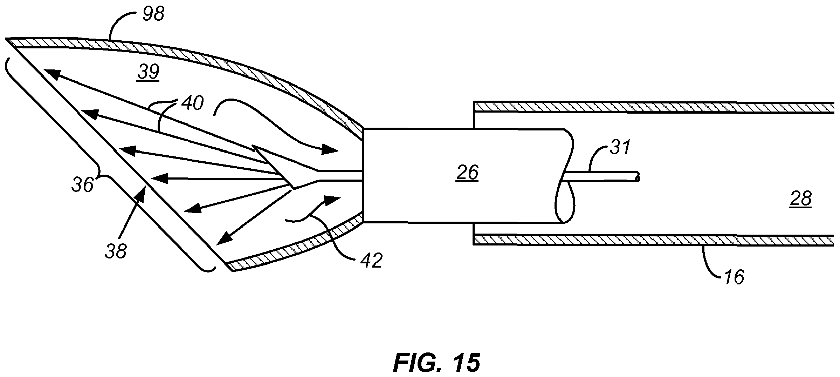

Fig. 15 shows a further example of a balloon type focal ablation system comprising a balloon type cap.

### DETAILED DESCRIPTION

The following description will typically be with reference to specific structural embodiments and methods. It is to be understood that there is no intention to limit the invention to the specifically disclosed embodiments but that the invention may be practiced using other features, elements and embodiments. Preferred embodiments are described to illustrate the present invention, not to limit its scope, which is defined by the claims. Those of ordinary skill in the art will recognize a variety of equivalent variations on the description that follows. Like elements in various embodiments are commonly referred to with like reference numerals.

The control of three primary factors is necessary for repeatable cryoablation via evaporative cooling. These factors are evaporation temperature of the cryogen, the mass flow rate of the cryogen / surface area, and the amount of time that the cryogen is applied. The described assemblies directly address two of these factors. (1) Evaporation temperature of the cryogen is set by controlling the evaporation pressure. The evaporation pressure can be controlled by appropriately sizing the cryogenic refrigerant delivery and exhaust lumens. (2) The mass flow rate / surface area can be controlled by appropriately sizing the cryogenic refrigerant delivery lumen (to control mass flow rate) and defining a fixed treatment area either by physically defining a treatment area or by controlling distribution of the cryogen delivery onto a treatment surface.

Fig. 1A is an overall view showing a generalized example of a focal ablation system 10 including broadly an endoscope 12 and a focal ablation assembly 14. Endoscope 12 includes an endoscopic tube 16 having an accessory channel port 18 at a proximal end 20 of endoscopic tube 16 and a cap 22 at the distal end 24 of endoscopic tube 16. Endoscope 12 may be a conventional endoscope such as Olympus GIF-140 or GIF-Q160Z, that connects to an image processor 19, which then displays the image on monitor 21. Fig. 1B is similar to Fig. 1A but shows cap 22 mounted to the distal end 34 of cryogenic catheter 26 instead of endoscopic tube 16.

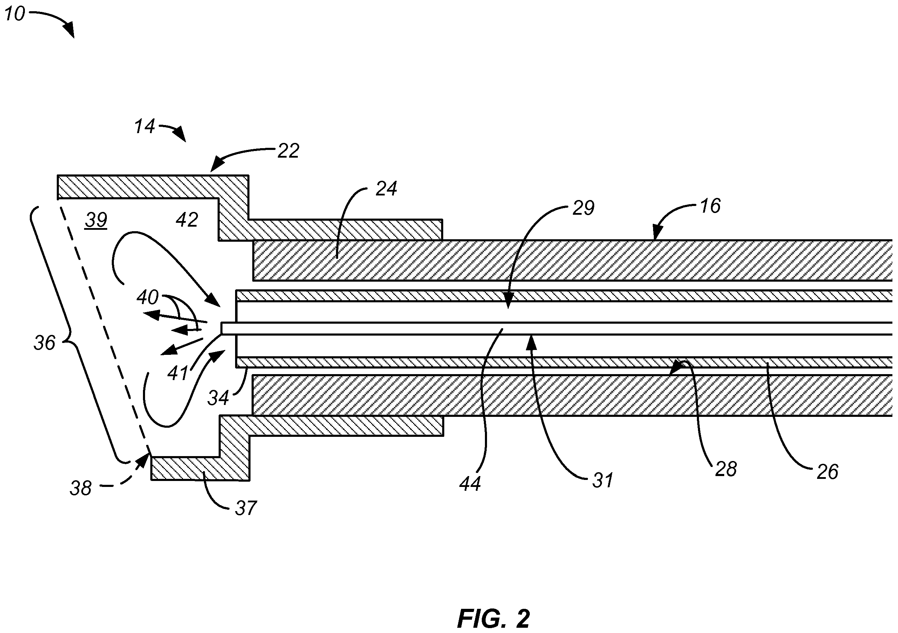

Figs. 2-12 are directed to a first type of a focal ablation system 10 .

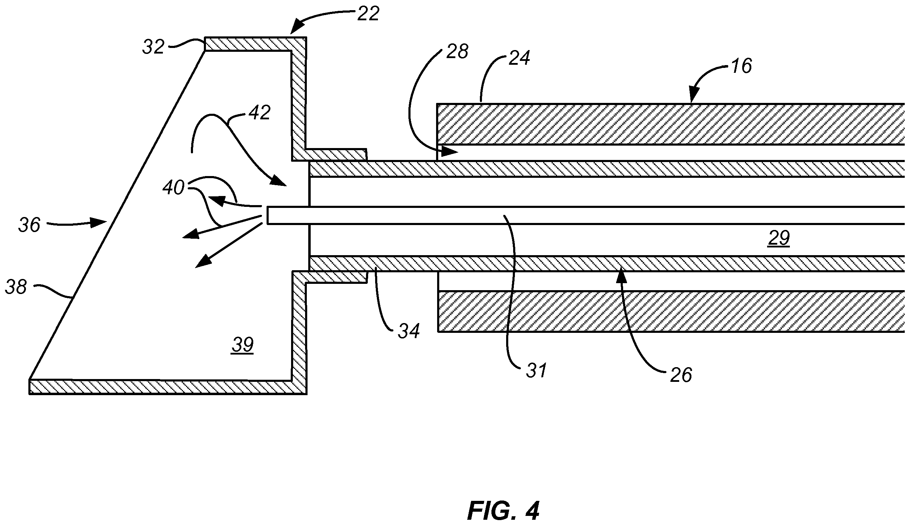

Focal ablation assembly 14, see Figs. 1 and 2, includes a cap 22 mounted to the distal end 24 of an endoscopic tube 16 and a cryogenic catheter 26 passing through the channel 28 of endoscopic tube 16. Focal ablation assembly 14 also includes a cryoablation controller 25 at the proximal end of 27 of cryogenic catheter 26. In some examples, described below with reference to Figs. 1B and 4, cap 22 can be mounted to the distal end 34 of cryogenic catheter 26. Cap 22 in Fig. 2 has a generally cylindrical cross-sectional shape and defines a centerline 30. Cap 22 has a distal end 32 extending distally of the distal end 24 of endoscopic tube 16 and the distal end 34 of cryogenic catheter 26. In this example cap 22 is oriented obliquely to centerline 30 to facilitate tissue apposition. Cryogenic catheter 26 defines a catheter lumen 29 through which a delivery catheter 31 passes. As is discussed in more detail below, distal end 32 of cap 22 will be placed against tissue at the target site to be treated.

Cap 22 is preferably of a clear, semi-rigid, soft, flexible material, or a combination of materials, such as a polymer material, so to substantially maintain its shape during use while not causing tissue trauma. Examples of the material for 22 include silicone, polyurethane, polyvinyl chloride, and C-Flex®, a thermoplastic elastomer, specifically styrene-ethylene-butylene modified block copolymer with silicone oil. Cap 22 may be manufactured by, for example, injection molding, casting, or thermoforming. Distal end 32 of cap 22 defines a thermally conductive therapeutic region 36 having a typical cross-sectional area of 0.5 cm² to 3.0 cm². In this example therapeutic region 36 is covered by a cover 38 of a thin transparent polymer, such as polyurethane having a thickness of typically less than 0.05 mm (0.002 inch). In this way the liquid refrigerant 40 passing through the delivery lumen of delivery catheter 31 and out through the exit opening 41 of delivery catheter 31 does not contact tissue outside the target site but rather heat is removed from the tissue at the target site as the liquid refrigerant evaporates while in contact with cover 38. Although polyurethane may not be considered to be highly thermally conductive, the thinness of cover 38 allows cover 38 to provide effectively no thermal insulation between the evaporating liquid refrigerant and the target tissue. In addition, it is preferable that cover 38 be transparent or at least translucent so that the physician can see what is happening to the tissue at the target site. The selection of the size of therapeutic region 36 is typically chosen according to the size of the treatment site or the desired mass flow rate/surface area for refrigerant 40, or both. Evaporated refrigerant 42 passes out of cap volume 39 through catheter lumen 29 between delivery catheter 31 and the inner wall of cryogenic catheter 26.

Cover 38 is stated to provide effectively no thermal insulation between the evaporating liquid refrigerant in the target tissue. In this application the phrase effectively no thermal insulation is meant to mean that tissue necrosis can occur at the target site upon the application of a cryogenically ablative liquid refrigerant, such as nitrous oxide (N₂O), to the surface of cover 38.

Cryogenic catheter 26 may be sized appropriately for introduction through, for example, 2.0 mm, 2.8 mm or 3.7 mm diameter instrument channels 28. Cryogenic catheter 26 may be constructed from materials such as PEBAX or nylon. Delivery catheter 31 may be constructed from a rigid polymer such as polyimide or a metal such as stainless steel, sufficient to withstand internal pressure approaching 69 Bar (1000 psig). The diameter of delivery lumen 44 defined by delivery catheter 31 is typically in the range of 0.15 mm (.006 inch) to 0.30 mm (.012 inch). The diameter of delivery lumen 44 can be chosen according to the desired mass flow rate/surface area for refrigerant 40 contacting therapeutic region 36.

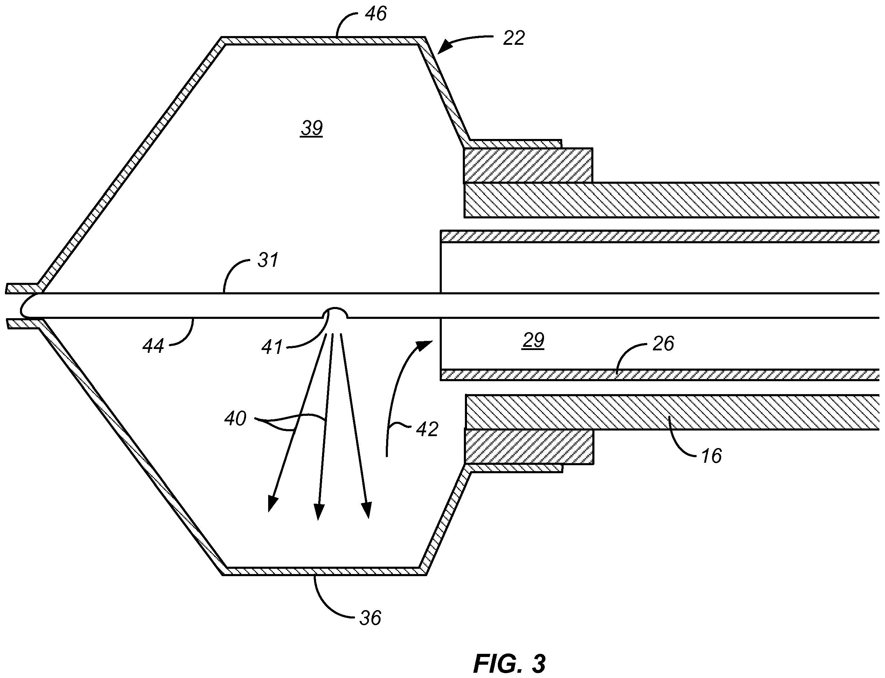

Fig. 3 is a view similar to that of Fig. 2 of an alternative example of the focal ablation system 10 of Fig. 1 in which the therapeutic region 36 is along a sidewall 35 of the cap. Delivery catheter 31 extends completely through cap volume 39 and is secured to a distal end of cap 22. Delivery catheter 31 has a laterally oriented exit opening 41 positioned opposite the therapeutic region 36 along sidewall 46 of cap 22. This feature permits liquid refrigerant 40 to be directed against sidewall 46 at therapeutic region 36 so the target tissue at the target treatment site 78 against which therapeutic region 36 is pressing, see Fig. 12, can be thermally ablated due to the low temperature of the liquid refrigerant.

Fig. 4 is a view similar to that of Fig. 2 wherein the cap 22 is mounted to the distal end 34 of the cryogenic catheter 26 instead of the endoscopic tube 16. See also Fig. 1B.

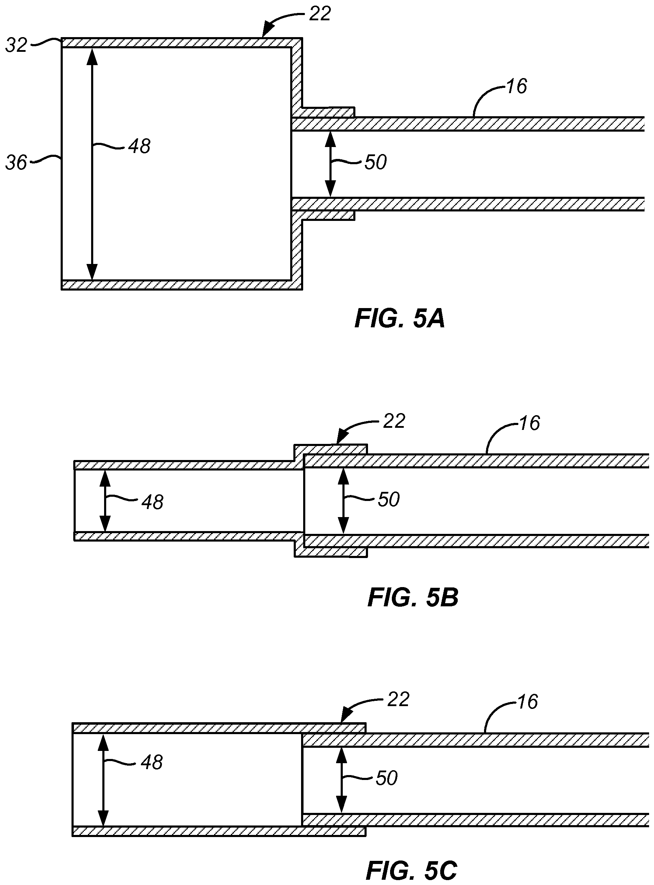

Figs. 5A-5C are simplified cross-sectional views of examples of three different caps 22 in which the cross-sectional area 48 of the therapeutic region 36 at the distal end 32 of each cap 22 is different while the cross-sectional area 50 of the endoscopic tube 16 to which it is mounted remains the same. This concept is used to allow the physician to choose the appropriately sized cap 22 according to the size of the target site, or the mass flow rate/surface area of refrigerant 40, or both.

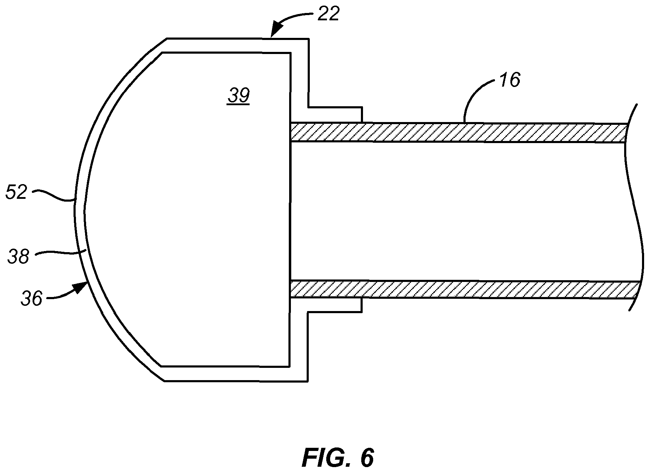

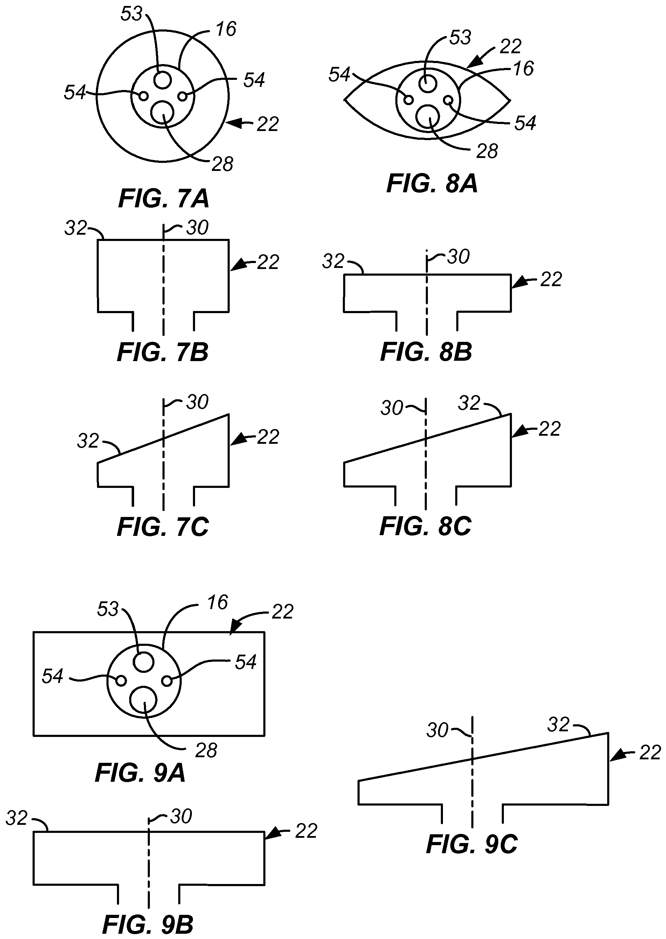

Fig. 6 illustrates an example in which the therapeutic region 36 has a convex outer surface 52. This configuration may be useful to improve the amount of contact between the therapeutic surface of the cap and the target tissue. Figs. 7A, 8A and 9A are simplified end views showing caps 22 having round, generally oval and rectangular cross-sectional shapes, respectively. Each cap 22 is mounted to the distal end 24 of an endoscopic tube 16. Each figure illustrates a conventional endoscope 12 having, in this example, a camera 53, two lights 54 for illumination and a working channel 28.

Figs. 7B, 8B and 9B show the caps 22 of Figs. 7A, 8A and 9A having distal ends 32 oriented perpendicular to the centerlines 30 of the respective caps. Figs. 7C, 8C and 9C show the caps 22 of Figs. 7A, 8A and 9A having distal ends 32 oriented obliquely to the centerlines 30 of the respective caps.

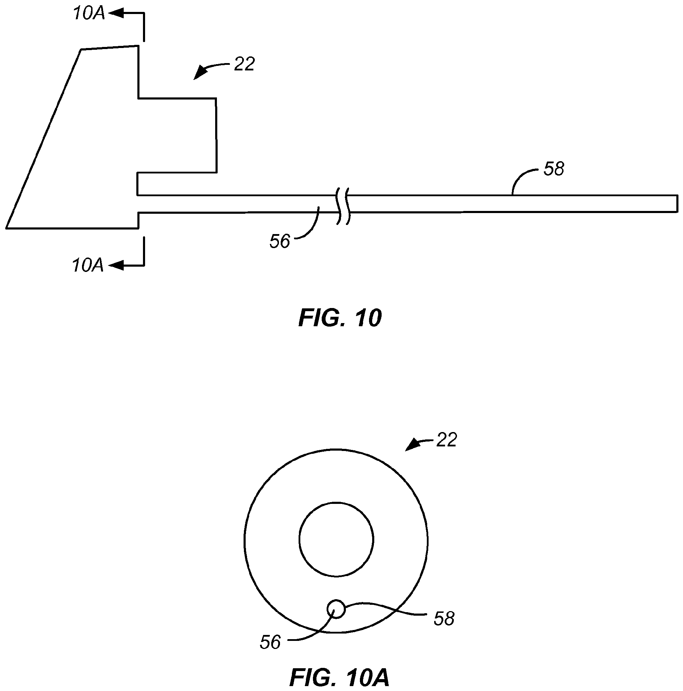

Fig. 10 shows an example of cap 22 similar to the cap 22 of Fig. 2 but in which an exhaust lumen 56 is defined by a coaxial extension 58 of cap 22. Extension 58 will lie generally parallel to the endoscopic tube 16. This permits and a larger cross-sectional area for the exhaust lumen than would be typically available if the exhaust lumen was defined by an accessory channel of endoscopic tube 16. Exhaust lumen 56 is typically in the range of 2-5 mm in diameter. The cross-sectional areas provided by catheter lumen 29 when used as exhaust lumens in the above examples are typically of a similar size.

Fig. 11 shows another example in which an elastomeric balloon 60 is mounted to the distal end 34 of the cryogenic catheter 26. Balloon 60 expands within the cap volume 39 of cap 22 with a portion 62 of the balloon forming the cover 38 along the therapeutic region 36 at the distal end 32 of the cap. A vent hole 63 is formed in the sidewall 35 of cap 22 to facilitate the expansion of balloon 60.

In use, the physician will typically select a cap 22 having the appropriate size and shape for the particular target treatment site 78. The size of therapeutic region 36 may also be chosen according to the desired mass flow rate/surface area for refrigerant 40. Assuming the focal ablation system 10 of Fig. 2 is being used, cap 22 can be installed on the distal end 24 of endoscopic tube 16. Cryogenic catheter 26, typically with delivery catheter 31 therein, can be placed through accessory port 18 of the endoscope 12 and passes through channel 28 of the endoscopic tube 16 until the distal end 34 of cryogenic catheter 26 is at cap volume 39. Note that the installation of cap 16 could occur after positioning cryogenic catheter 26 within endoscope 12. The distal portion of focal ablation system 10 is placed in the patient so that region 36 is properly positioned at the target treatment site 78. The refrigerant from cryoablation controller 25 is then directed through delivery catheter 31 and against cover 38 at to the target treatment site 78. The evaporation of the refrigerant on cover 38 lowers the temperature of the tissue at the target treatment site enough to cause necrosis of the tissue. The evaporated refrigerant 42 passes out of cap volume 39 through catheter lumen 29.

Figs. 12-15 show balloon type of focal ablation systems, somewhat different from the focal ablation system of Figs. 1-11.

Figs. 12 and 12A are simplified side and end cross-sectional views of a balloon type focal ablation system 70 in which a balloon type focal ablation assembly 72 is used with an endoscopic tube 16 of an endoscope 12. A balloon 74 is mounted to the distal end 34 of cryogenic catheter 26. The cryogenic catheter 26 extends through the channel 28 of endoscopic tube 16. The therapeutic region 36 is along a sidewall 76 of balloon 74. In this example cryogenic catheter 26 has a dogleg shape distal portion to facilitate placement of therapeutic region 36 along sidewall 76 and against a target treatment site 78 of the body structure 80. Delivery catheter 82 passes through lumen 29 with its distal end 84 secured to the distal end 86 of balloon 74. Delivery catheter 82 has, in this example, a number of laterally directed exit openings 41 acting as delivery ports to direct liquid refrigerant 40 into the interior 85 of balloon 74 and against sidewall 76 of balloon 74 at target site 78. The size, number and positions of openings 41 can be chosen according to the size of the target treatment site 78 and desired mass flow rate/surface area for refrigerant 40. Fig. 12A shows the use of reinforcing elements 88, such as nitinol support wires, within the balloon 74 to cause the balloon to have a flattened or oval cross-sectional shape to better conform to the shape of the body structure 80. The use of reinforcing elements 88 also helps to maintain balloon 74 in direct contact with the body structure during the procedure.

Figs. 13 and 13A are simplified side and end cross-sectional views of a focal ablation balloon 74 and reinforcing elements 88 similar to those of Figs. 12 and 12A.

Fig. 14 illustrates another example of a balloon type focal ablation system 70 in which the distal end of the reinforcing element 88 extends through the delivery catheter 31. The distal end 90 of reinforcing element 88 is secured to the central portion of the working region 36 of balloon 74 in the following manner. Balloon 74 has a stem portion 92 at the center of therapeutic, working region 36 extending inwardly into the volume 93 defined by the balloon. A thermally conductive filler material 94 is used to secure distal end 90 of reinforcing element 88 to stem portion 92. As indicated by liquid refrigerant arrows 40, liquid refrigerant is directed toward the center of working region 36 surrounding stem portion 92.

Fig. 15 shows an example of a balloon type focal ablation system comprising a balloon type cap 98, which is not generally rigid as caps 22 in the examples above Figs. 1-11. Rather, cap 98 is typically made of one or more materials similar to those used with balloon 74 and includes reinforcing elements 88, not shown in Fig. 15, which may be made of, for example, a metal, such as nitinol, or of the same material as the rest of cap 98. Cap 98 also includes a cover 38, typically made of a thin film of silicone, polyurethane, or PET. As with the balloon type focal ablation systems 70 of Figs. 12-14, the full expansion of cap 98 is typically the result of reinforcing elements 88 and the internal pressure created by the vaporization of refrigerant 40 and expansion due to the creation of exhaust gas 42.

In the use of the focal ablation system 70 of Figs. 12-13A, the physician will typically select a focal ablation assembly 72 including a balloon 74 having the appropriate size and shape for the particular target treatment site 78. Cryogenic catheter 26, typically with delivery catheter 82 therein and the balloon 74 at the distal end 34, can be placed through accessory port 18 of the endoscope 12 and pass through channel 28 of the endoscopic tube 16 until sidewall 76 of balloon 74 is adjacent to therapeutic region 36 of body structure 80. The refrigerant 40 from cryoablation controller 25 is then directed through delivery catheter 82 and against balloon sidewall 76 at to the target treatment site 78. The evaporation of the refrigerant on sidewall 76 of balloon 74 lowers the temperature of the tissue at the target treatment site 78 enough to cause necrosis of the target tissue. The evaporated refrigerant 42 passes out of the interior of balloon 74 through catheter lumen 29. The use of the examples of Figs. 14 and 15 are carried out in similar manners with the working region 36 positioned at target treatment site 78

The above descriptions may have used terms such as above, below, top, bottom, over, under, et cetera. These terms may be used in the description and claims to aid understanding of the invention and not used in a limiting sense.

While the present invention is disclosed by reference to the preferred embodiments and examples detailed above, it is to be understood that these examples are intended in an illustrative rather than in a limiting sense. It is contemplated that modifications and combinations will occur to those skilled in the art, which modifications and combinations will be within the scope of the following claims. For example, cryogenic catheter 26 may also contain features related to measuring the performance of the system for either safety or efficacy reasons. Examples of these features include a pressure sensing lumen for monitoring pressure in the volumes 39, 93, and a temperature sensing device (e.g. thermistor or thermocouple) for monitoring temperature in the cap 22, especially at working region 36.

## Claims

1. A focal ablation assembly (14), for use with an endoscope (12) comprising an endoscopic tube having proximal and distal ends and defining a channel (28) extending between the proximal and distal ends, the focal ablation assembly comprising:
a cryogenic catheter (26) placeable within the channel (28), the cryogenic catheter defining a catheter lumen and having a distal end placeable at the distal end of the endoscopic tube;
a cap (22) mountable to at least one of the distal end of the endoscopic tube and the distal end of the cryogenic catheter (26), the cap extending distally of both of the distal ends;
the cap (22) comprising a material which substantially maintains its shape during use while not causing tissue trauma;
the cap (22) defining a limited area therapeutic region (36);
the cap (22) defining a cap volume (39) defined by a wall of the cap and the therapeutic region (36); and
the therapeutic region being a chosen one of:
an open therapeutic region; and
a covered, optically transparent therapeutic region providing effectively no thermal insulation, **characterized in that** the assembly further comprises:
a delivery catheter (31) extending along the channel and having a distal portion fluidly coupled to the cap volume (39), whereby refrigerant can be introduced into the cap volume (39) and towards the therapeutic region (36) by the delivery catheter (31); and
a balloon (60) extending from the distal end of the cryogenic catheter (26) within the therapeutic region (36) so that the refrigerant can be introduced into the balloon within the cap volume by the delivery catheter (31).

2. The assembly according to claim 1, wherein the cap (22) has a sidewall and the therapeutic region (36) is at the sidewall.

## Patentansprüche

1. Fokale Ablationsanordnung (14) zur Verwendung mit einem Endoskop (12), das eine endoskopische Röhre mit proximalen und distalen Enden umfasst, und einen Kanals (28) definiert, der sich zwischen dem proximalen und dem distalen Ende erstreckt, wobei die fokale Ablationsanordnung Folgendes umfasst:
einen kryogenen Katheter (26), der in dem Kanal (28) angeordnet werden kann, wobei der kryogene Katheter ein Katheterlumen definiert und ein distales Ende aufweist, das am distalen Ende der endoskopischen Röhre angeordnet werden kann;
eine Kappe (22), die an mindestens einem von dem distalen Ende der endoskopischen Röhre und dem distalen Ende des kryogenen Katheters (26) angebracht werden kann, wobei sich die Kappe distal von beiden der distalen Enden erstreckt;
wobei die Kappe (22) ein Material umfasst, das während der Verwendung im Wesentlichen seine Form beibehält, während es kein Gewebetrauma verursacht;
wobei die Kappe (22) eine begrenzte therapeutische Zone (36) definiert;
wobei die Kappe (22) ein Kappenvolumen (39) definiert, das durch eine Wand der Kappe und den therapeutischen Bereich (36) definiert ist; und
wobei der therapeutische Bereich aus Folgendem ausgewählt wird:
einem offenen therapeutischen Bereich ; und
einem bedeckten, optisch transparenten therapeutischen Bereich, der effektiv keine Wärmeisolierung bereitstellt, **dadurch gekennzeichnet, dass** die Anordnung ferner Folgendes umfasst:
einen Zuführungskatheter (31), der sich entlang des Kanals erstreckt und einen distalen Abschnitt aufweist, der mit dem Kappenvolumen (39) fluidisch verbunden ist, wobei durch den Zuführungskatheter (31) Kühlmittel in das Kappenvolumen (39) und in Richtung des therapeutischen Bereichs (36) eingeführt werden kann; und
ein Ballon (60), der sich von dem distalen Ende des kryogenen Katheters (26) innerhalb des therapeutischen Bereichs (36) erstreckt, so dass das Kühlmittel durch den Zuführungskatheter (31) in den Ballon innerhalb des Kappenvolumens eingeführt werden kann.

2. Anordnung nach Anspruch 1, wobei die Kappe (22) eine Seitenwand aufweist und der therapeutische Bereich (36) an der Seitenwand angeordnet ist.

## Revendications

1. Ensemble d'ablation focale (14), pour une utilisation avec un endoscope (12) comprenant un tube endoscopique ayant des extrémités proximale et distale et définissant un canal (28) s'étendant entre les extrémités proximale et distale, l'ensemble d'ablation focale comprenant
un cathéter cryogénique (26) pouvant être placé au sein du canal (28), le cathéter cryogénique définissant une lumière de cathéter et ayant une extrémité distale pouvant être placée au niveau de l'extrémité distale du tube endoscopique ;
un capuchon (22) pouvant être monté sur au moins une extrémité parmi l'extrémité distale du tube endoscopique et l'extrémité distale du cathéter cryogénique (26), le capuchon s'étendant de manière distale des deux extrémités distales ;
le capuchon (22) comprenant un matériau qui garde essentiellement sa forme pendant l'utilisation sans provoquer de traumatisme tissulaire ;
le capuchon (22) définissant une région thérapeutique à superficie limitée (36) ;
le capuchon (22) définissant un volume de capuchon (39) défini par une paroi du capuchon et la région thérapeutique (36) ; et
la région thérapeutique étant une région choisie parmi :
une région thérapeutique ouverte ; et
une région thérapeutique transparente optiquement, couverte, ne fournissant effectivement aucune isolation thermique, **caractérisé en ce que** l'ensemble comprend en outre
un cathéter de distribution (31) s'étendant le long du canal et ayant une partie distale couplée de manière fluidique au volume de capuchon (39), par quoi du réfrigérant peut être introduit jusque dans le volume de capuchon (39) et vers la région thérapeutique (36) par le cathéter de distribution (31) ; et
un ballonnet (60) s'étendant de l'extrémité distale du cathéter cryogénique (26) au sein de la région thérapeutique (36) de sorte que le réfrigérant peut être introduit jusque dans le ballonnet au sein du volume de bouchon par le cathéter de distribution (31).

2. Assemblage selon la revendication 1, dans lequel le capuchon (22) a une paroi latérale et la région thérapeutique (36) est au niveau de la paroi latérale.
